Europäisches Patentamt

(19) European Patent Office

Office européen des brevets

(11) Publication number: **0 038 265**
**B1**

(12) **EUROPEAN PATENT SPECIFICATION**

(45) Date of publication of patent specification: **02.10.85**

(51) Int. Cl.⁴: **A 61 K 39/09, C 12 P 19/26**

(21) Application number: **81400585.6**

(22) Date of filing: **13.04.81**

(54) Group B streptococcal capsular polysaccharides.

(30) Priority: **14.04.80 US 140031**

(43) Date of publication of application:
**21.10.81 Bulletin 81/42**

(45) Publication of the grant of the patent:
**02.10.85 Bulletin 85/40**

(84) Designated Contracting States:
**AT BE CH DE FR GB IT LI LU NL SE**

(56) References cited:
**EP-A-0 008 969**

**CHEMICAL ABSTRACTS, vol. 93, no. 19, November 10, 1980 page 476, abstract 184091e Columbus, Ohio, USA H.J. JENNINGS et al. "Structure and serology of the native polysaccharide antigen of type Ia group B Streptococcus"**
**CHEMICAL ABSTRACTS, vol. 92, no. 23, June 9, 1980, page 500, abstract 196165 Columbus, Ohio, USA H.J. JENNINGS et al. "Structural determination and serology of the native polysaccharide antigen of type-III group B Streptococcus"**

(73) Proprietor: **MERCK & CO. INC.**
**126, East Lincoln Avenue P.O. Box 2000**
**Rahway New Jersey 07065 (US)**

(72) Inventor: **Carlo, Dennis J.**
**733 Schoolhouse Lane**
**Bound Brook New Jersey 08805 (US)**
Inventor: **Nollstadt, Karl H.**
**91 Stonehenge Terrace**
**Clark New Jersey 07066 (US)**
Inventor: **Stoudt, Thomas H.**
**857 Village Green**
**Westfield New Jersey 07090 (US)**
Inventor: **Maigetter, Robert z.**
**39 Shadyside Avenue**
**Summit New Jersey 07901 (US)**

(74) Representative: **Martin, Jean-Jacques et al**
**Cabinet REGIMBEAU 26, Avenue Kléber**
**F-75116 Paris (FR)**

(56) References cited:

**CHEMICAL ABSTRACTS, vol. 94, no. 9, March 2, 1981, page 541, abstract 63452k Columbus, Ohio, USA T.I. DORAN et al. "Extracellular antigens of serotype III group B streptococci"**
**CHEMICAL ABSTRACTS, vol. 90, no. 15, April 9. 1979, page 471, abstract 119515v Columbus, Ohio, USA J.Y. TAI et al. "Isolation of type-specific polysaccharide antigen from group B type Ib streptococci"**
**CHEMICAL ABSTRACTS, vol. 93, no. 7, August 18, 1980, page 700, abstract 68332k Columbus, Ohio, USA J.Y. TAI et al. "Isolation of type-specific polysaccharide antigen from group-B type.Ib streptococci"**

**Description**

Background of the invention

Group B *Streptococcus (Streptococcus agalactiae)* has recently been identified as the leading cause of meningitis among neonates and young infants. A conservative estimation [C. J. Baker, *J. Infectious Diseases*, *136*, No. 1, 137 (1977)] indicates that about 12,000 to 15,000 infants will be affected annually. Half of them will die. Those who survive may develop fatal neurological sequelae that often progress too far to be arrested by antibiotic treatments. For this reason, the immunological, preventive approach as embodied in vaccination of Group B streptococcal type-specific polysaccharides has evolved as one of the most important aspects for future control of the disease. In addition, antibody specific to type $I_a$ *Streptococcus agalactiae* has recently been found in the glands of bovine immunized with killed or live vaccine, indicating that Group B streptococcal type-specific polysaccharides may be important in vaccination against mastitis in dairy mammals, for example, dairy cattle.

Group B streptococcal antigens are generally classified into five serotypes ($I_a$, $I_b$, $I_c$, II and III) based on capillary precipitin tests with hydrochloric acid-extracted antigens and type-specific hyperimmune rabbit antisera.

Although later studies have shown chemical compositions indicating structures quite different from those reported originally, the same serological classification, i.e., $I_a$, $I_b$, $I_c$, II and III for streptococcal polysaccharides, are still being followed. For example, type-specific polysaccharides isolated *via* HCl-treatment of whole organisms was reported to contain rhamnose, glucosamine, and galactose, while type-specific polysaccharides prepared by TCA (trichloroacetic acid) extraction contained an additional antigenic determinant, sialic acid. Furthermore, structural difference may also arise from variation of fermentation conditions. Thus Group B Streptococcal polysaccharides isolated by identical methods from organisms grown in the presence of excess glucose has been found to contain no rhamnose as previously reported.

Such polysaccharides as type-specific polysaccharide antigen from group B Type Ib Streptococci are mentioned in J. Exp. Med. 1979, 148(1), 58—66 and Pathog. Streptococci Proc. Int. Symp. 7th 1978, (publ. 1979), 154—5, as antigen of type III in Can. J. Biochem. 1980, 58(2), 112—20 and Infect. Immun. 1980 30(3), 890—3, and as polysaccharide antigen of type Ia in Proc. Natl. Acad. Sci. USA, 1980, 77(5), 2931—5.

The present invention relates to a process for the preparation of Group B streptococcal polysaccharide types $I_a$, $I_b$, II and III, all of which contain galactose, glucosamine, glucose, and sialic acid. They are structurally distinguishable from those reported earlier as a result of variation in fermentation conditions, methods of isolation and purification.

Accordingly, it is an object of the present invention to provide highly purified antigenic Group B streptococcal polysaccharide types $I_a$, $I_b$, II and III which can be used (1) to provide a vaccine for neonates or infants against Group B *Streptococcus* infections, for example, meningitis, containing at least one of the novel Group B streptococcal polysaccharides; (2) to provide a method for the prevention of neonatal diseases induced by Group B *Streptococcus* by vaccination of pregnant women or women of childbearing age: (3) to provide a vaccine containing one or more of the type-specific Group B streptococcal polysaccharides for protection against mastitis in dairy mammals; (4) to provide a method for protection against Group B *Streptococcus* infections by passive vaccination of patients with impaired immune system; and (5) to provide a method for protection against mastitis in dairy mammals by passive vaccination.

Detailed description of the invention

Antigenic Group B streptococcal polysaccharide types $I_a$, $I_b$, II and III have been isolated having the specifications shown below in Table I based on dry weight analysis.

TABLE I

Specification of group B Streptococcus type-specific polysaccharides

| | $I_a$ | $I_b$ | II | III |
|---|---|---|---|---|
| $K_d$ | 0.3—0.4 | 0.3—0.4 | 0.3—0.4 | 0.5—0.6 |
| %PRT | 0.5 | 1.0—2.1 | 0.5 | 1.2—5.0 |
| %NA | 0.2 | 0.5 | 0.2 | 0.1—0.5 |
| %P | 0.1—0.5 | 0.1—0.5 | 0.1—0.5 | 0.5 |
| %HXA | 13.7—22.5 | 12.0—18.0 | 10.0—15.8 | 20.0—27.0 |
| %OAC | 0.5 | 0.5—1.0 | 1.0 | 0.5 |
| %HEX | 35.0—40.0 | 50.0—62.5 | 35.0—45.3 | 45.0—55.0 |
| %GLU | 14.1—20.7 | 30.0—41.6 | 15.0—22.1 | 10.0—17.4 |
| %SA | 25.0—30.0 | 7.0—16.6 | 20.6—25.0 | 25.0—35.0 |
| %MP | 0.5—1.0 | 1.4 | 2.0—5.0 | 0.5—1.0 |

$K_d$=partition coefficient in Sepharose 4B gel chromatography
PRT=Protein
NA=Nucleic Acid
P=Phosphorus
HXA=Hexosamines (glucosamine)
OAC=O-acetates
HEX=Hexoses (including galactose and glucose in the ratio of 2:1)
GLU=Glucose
SA=Sialic Acid
MP=Methylpentose

The most purified antigens prepared by the process of this invention have the following analyses. In all cases, the hexosamine is glucosamine and the hexoses are galactose and glucose. $K_D$ values are as determined on Sepharose® 4B by gel filtration.

Type $I_a$ (product No. 11059—113) 42.9% hexose, 27.6% hexosamine, 29.3% sialic acid; $K_D$=0.22
Type $I_b$ (product No. 11059—193) 42.6% hexose, 18.3% hexosamine, 26.8% sialic acid, $K_D$=0.35
Type II (product No. 11059—176) 50.9% hexose, 14.2% hexosamine, 23.9% sialic acid, $K_D$=0.44
Type III (product No. 11059—179) 39.9% hexose, 23.3% hexosamine, 28.7% sialic acid, $K_D$=0.59

These type-specific antigens can therefore be defined as consisting essentially of polysaccharides of varying composition and molecular weights.

Type $I_a$ is composed of hexose, glucosamine, and sialic acid in the approximate molar ratio 3:1:1, having a molecular weight of about $0.8 \times 10^6$ daltons.

Type $I_b$ contains the same components in the approximate molar ratio 3:1:1, having a molecular weight of about $0.5 \times 10^6$ daltons.

Type II contains the same components in the approximate molar ratio 5:1:1, having a molecular weight of about $0.5 \times 10^6$ daltons.

Type III contains the same components in the approximate molar ratio 3:1:1, having a molecular weight of about $0.15 \times 10^6$ daltons.

The purified type-specific polysaccharides be they type $I_a$, $I_b$, II or III, are composed of galactose, glucosamine, glucose and sialic acid. Very small amounts of protein, nucleic acid, phosphorus, uronic acid and acetate are also present.

The polysaccharides can be isolated from any group B type-specific strain, but preferably from the following strains which are on unrestricted deposit in the American Culture Collections (ATCC).

| Type | Merck No. | ATCC No. |
|------|-----------|----------|
| I$_a$ | MB-4052 | 31,574 |
| I$_b$ | MB-4053 | 31,575 |
| II | MB-4055 | 31,576 |
| III | MB-4082 | 31,577 |
|  | MB-4316 (M 732) | 31,475 |

The most preferable strain for type III is M-732

According to the invention the preparation of antigenic polysaccharides comprises:

a) growing the Group B *Streptococcus* bacteria types I$_a$, I$_b$, II, or III in a high glucose, soy bean and yeast extract fermentation medium;

b) separating the cell paste from the liquid medium;

c) treating the liquid medium from step b with a strong ionic salt and a water-miscible lower alkanol to precipitate impurities or alternatively digesting the cell paste with an enzyme to obtain a liquid extract;

d) precipitating the crude polysaccharide from the treated liquid medium, or if desired, the liquid extract from digested cell paste with a sufficient amount of a water-miscible lower alkanol;

e) suspending the crude polysaccharide in deionized water and adding thereto sufficient cationic detergent to precipitate the polysaccharide;

f) redissolving the polysaccharide in 15% (wt/wt) sodium acetate aqueous solution;

g) precipitating the semi-purified polysaccharide from the solution with alcohol and, optionally, digesting the resulting precipitate with a proteolytic enzyme, followed by precipitating the enzyme-treated polysaccharide with a sufficient amount of water-miscible alkanol; and

h) eluting the resolubilized semi-purified polysaccharide of step g in a liquid medium through a gel column to obtain the purified polysaccharide.

Inoculation of the bacteria is accomplished by culturing a Group B *Streptococcus* type-specific strain, for example, MB-4055, (type II, ATCC No. 31576) in an inoculum medium such as Fluid Thioglycollate Medium (FTM). The FTM culture obtained is in turn used to inoculate a second inoculum medium which is free of animal protein and contains the necessary nutrients for the growth of the bacteria. For example, the preferred medium contains about 15 to 25 g per liter Hysoy® (Humko Sheffield), about 5 to 15 g per liter Amberex® 1003 (Amber), about 2.5 to 7.5 g per liter sodium chloride or potassium chloride, about 1 to 5 g per liter potassium or sodium phosphate dibasic (K$_2$HPO$_4$ or Na$_2$HPO$_4$), about 0.01 g per liter phenol red and about 10 to 50 g per liter glucose in distilled water at pH 6 to 8, preferably 6.8 to 7.4. After a subsequent 9-liter inoculation, the culture which is assured of its purity according to conventional methods (see Example 1, infra), is inoculated for the large scale production fermentation. The production medium is essentially the same as the inoculum medium except that phenol red is displaced with 8% UCON® LB 625 solution (see Table II, for definition) to prevent foam formation.

The more preferable medium is the medium which contains about 20 g per liter HySoy®, about 5 g per liter sodium chloride, about 2.5 g per liter potassium phosphate dibasic, about 10 g per liter Amberex® 1003 and about 25 g per liter (about 2.5% by weight) glucose in apyrogenic water at pH 6.9 to 7.4.

More specifically, the most preferable medium for type III polysaccharide contains the same amount of ingredients as described above. However, a solution of HySoy® and Amberex® 1003 (yeast extract) in about 50 ml of distilled water is dialyzed first with a molecular weight cut-off at about 12,000 to 14,000 before the resulting dialysate is mixed with other components, diluted to about 750 to 1000 ml with apyrogenic water, and adjusted to pH 7.2 before sterilization.

Generally the fermentation is conducted at about 34 to 39°C, preferably at 37°C, until the fermentation is complete, usually from about 3 hours to about 48 hours. Under optimum conditions, the fermentation is over within 24 hours.

After the fermentation is complete, it is stopped by addition of about 0.5 to 2.0% by weight of phenol followed by collection of the cells *via* centrifugation or filtration, preferably by centrifugation. In most cases, the cell paste is discarded except where enzymatic digestion is used to prepare polysaccharide type . I$_a$ or I$_b$ from the cells (see Example 2, infra). The preferable method initially involves (1) treating the supernatant or filtrate with a strong ionic salt such as calcium chloride, sodium sulfate, sodium chloride, or potassium chloride, preferably calcium chloride; (2) diluting the solution with a lower alkanol such as methanol, ethanol, propanol, or butanol, preferably ethanol (20 to 50% by volume) to precipitate most of the inactive impurities such as nucleic acids; and (3) precipitating from the supernatant obtained above the desired crude type-specific polysaccharide by addition of a water-miscible lower alkanol, preferably ethanol (35 to 75% by volume).

The most preferable conditions for removal of impurities and precipitation of the crude type-specific polysaccharides including types I$_a$, I$_b$, II and III are as follows:

| Removal of impurities | Precipitation of crude polysaccharide |
|---|---|
| 0.1M CaCl$_2$ in 40—44% by volume aqueous ethanol | CaCl$_2$ (ca. 0.07M) in 44—61% by volume aqueous ethanol |

The polysaccharides are then further purified by the formation of a complex with a cationic detegent, preferably cetavlon (hexadecyltrimethyl ammonium bromide). Under this procedure, the concentrated, combined active fractions are treated with a sufficient amount of cetavlon to form a gel-like precipitate which in turn is dissolved in a minimum volume of 15% by weight aqueous sodium acetate. About 2 to 4 volumes of water-miscible alcohol, preferably ethanol are added to precipitate the purified polysaccharide.

The purified polysaccharide is then further purified by (1) dissolution in a buffer solution and (2) elution through a gel column. Active fractions are pooled and concentrated. The resulting retentate is freeze-dried to afford the final product.

After the second alcohol precipitation, the product is optionally suspended in an appropriate amount of an aqueous solution of an inert salt, for example, sodium acetate or ammonium acetate. A sufficient amount of trypsin (about 1 mg to 10 mg/100 ml of the suspension) is added and the mixture is incubated at about 34°C to 38°C, preferably at 37°C, for about 0.5 to 2 hours or until the digestion is substantially complete. During the incubation, the pH is maintained at about 8.0 to about 8.5. Semipurified polysaccharides are subsequently obtained by a further alcohol precipitation.

A vaccine for humans may be prepared by incorporating an effective amount of one or more purified Group B *Streptococcus* type-specific polysaccharides into a suitable physiologically acceptable medium, for example, saline, water, or phosphate buffered saline.

The dosage of a monovalent vaccine and frequency of administration vary according to the age and physical condition of a patient and is up to the judgement of the clinician.

Usually the dose varies from about 25 µg to about 250 µg of the polysaccharide in about 0.2 ml to 1.0 ml sterile saline or the like. Generally, one or two subcutaneous administrations are sufficient to provide adequate protection against Group B *Streptococcus* infections.

In some cases, in the judgement of the clinician, it may be advantageous to prepare a polyvalent vaccine containing an effective amount of two or more selected types of Group B streptococcal type-specific polysaccharides, for example, a trivalent vaccine containing types I$_a$, II and III or a tetravalent vaccine containing types I$_a$, I$_b$, II and III. Dosage and administration of a polyvalent vaccine is substantially similar to that of a monovalent vaccine subject to the judgement of the clinician. Generally, the amount of each polysaccharides is from about 25 µg to 250 µg per dose.

Similar dosages are used in administrating a vaccine against mastitis in dairy mammals especially dairy cattle. However, an adjuvant—the role of which is to assure high immune response—is usually required. The adjuvant may be any compound or composition selected from the group consisting of aluminium hydroxide and aluminium phosphate; for example, ALHYDOGEL®, (Superfos Export Co., Copenhagen, Denmark).

Alternatively, protection against Group B *Streptococcus* induced infection can be established by passive vaccination, i.e., by giving preformed antibody or homologous γ-globulin from another individual of the same or a different species. The preformed antibody or γ-globulin used for passive vaccination is usually obtained from another individual who was vaccinated actively with the vaccines of the present invention.

The following examples illustrate the present invention.

Example 1
Fermentation of *Streptococcus* group B bacteria (non-dialyzed medium)
Step A: Culture

*Streptococcus* Group B type I$_a$, I$_b$, II, and III were received by Merck's Rahway Stock Culture Collection (RSCC) from Dr. R. Lancefield, Rockefeller University, New York, New York and were designated as Merck MB-4052, MB-4053, MB-4055 and MB-4082, respectively. The cultures were preserved as lyophilized cultures in the RSCC and have been deposited in the American Type Culture Collection (ATCC) and thereby assigned ATCC numbers 31574, 31575, 31576 and 31577, respectively. Another Type III culture, M-732 (ATCC No. 31,475), which is available on unrestricted deposit with the American Type Culture Collection, Rockville, Maryland, was designated as Merck MB-4316.

Step B: Inoculum development

A lyophilized "L" tube containing one of the cultures (type I$_a$) obtained from the RSCC was suspended in 1.0 ml of BBL Fluid Thioglycollate Medium (FTM, see Table I, 1, on pg. 17) and 1.0 ml of the resulting suspension was transferred into a test tube containing 9 ml of FTM and incubated at 37°C for 6 hours. After incubation the culture was examined microscopically and streaked on YED (see Table II, 2) plates to check for purity. Small colonies typical of Group B *Streptococcus* were observed on the YED plate; and numerous streptococcal chains typical of Group B *Streptococcus* were also observed microscopically.

Five ml of the FTM culture obtained above was used to inoculate 1 liter of inoculum medium (see Table

II, 3) in a 2-liter Erlenmeyer flask. The flask was incubated stationarily for about 9 to 12 hours at 37°C and about 4 to 12 hours at 4°C. The pH of the fermentation was adjusted periodically by the addition of 12% aqueous sodium bicarbonate. The 1 liter-fermentation utilized about 80 to 100 ml of the sodium bicarbonate solution.

Before the next inoculation, a sample was (1) applied on a YED plate to check its purity; (2) observed microscopically; and (3) examined for precipitin reaction with Group B streptococcal antiserum (see Table II, 6). Small colonies, typical of Group B *Streptococcus,* were viewed on the YED; the cells were streptococcal formation when examined microscopically; and a single precipitin reaction with Group B antiserum was observed on the Ouchterlony plates.

The culture which was assured of its purity was used to inoculate a 14-liter fermentor (MA 114—New Brunswick Scientific, Edison, New Jersey) containing 9 liters of inoculation medium (see Table II, 4). The batch was incubated at 37°C with mild agitation (100 rpm) without aeration. Throughout the cultivation, samples were examined for optical density (O.D.) and pH values. The final O.D. before inoculating the production stage was about 2.0 to 5.0. The pH was maintained at about 7.0 during fermentation by the periodical addition of 10% aqueous sodium hydroxide. After the final sodium hydroxide addition, the fermentation was terminated (total time 3 hrs.). A total of 150 ml of aqueous sodium hydroxide (10%) was utilized.

Before inoculating the production fermentor, samples again were taken for purity (YED), Group B specificity (precipitin reaction on Ouchterlony), and microscopic examination. Based upon these examinations, which revealed only streptococcal forms, the production-fermentor was inoculated as described in the next step.

Step C: Production of fermentation broth

Approximately 10 liters of the culture obtained from Step B was used to inoculate a 250-liter fermentor (FM 250, New Brunswick Scientific) containing 175 liters of production medium (see Table II, 5). The conditions for growth were 37°C, 100 rpm agitation, and no aeration. Throughout the cultivation the pH was adjusted to 7.0 with 10% aqueous sodium hydroxide and samples were taken at 2 hr. intervals for optical density (O.D.) and pH measurements. When the pH remained constant at about 7.0 without further additions of sodium hydroxide, the fermentation was stopped.

Prior to harvesting, the culture was plated on YED, examined microscopically, checked by Gram strain (see Table II, 7), and examined for group specificity by Ouchterlony reaction. There were small colonies on the YED plate; the cells were in streptococcal formation when observed under the microscope; the Gram stain was positive; and a single precipitin reaction occurred. These results indicated that the fermentation broth was ready for harvest and inactivation. It was inactivated *via* addition of phenol (1% by weight).

Following substantially the same procedure as described in Example 1, but substituting for the type $I_a$ culture used therein culture type $I_b$, II, or III, there was obtained the corresponding type $I_b$, type II or type III fermentation broth.

TABLE II
Definition

| 1. Fluid Thioglycollate Medium (FTM) | |
|---|---|
| Fluid thioglycollate powder | 29.5 g/liter |
| | |
| 2. YED Plates | |
| Amberex® 1003 (Amber) | 10 gm/liter |
| Dextrose | 10 gm/liter |
| Agar (Difco) | 20 gm/liter |
| | |
| 3. Inoculum Medium (2-Liter Flask) | |
| HySoy® (Humko Sheffield) | 20 gm |
| Amberex® 1003 (Amber) | 10 gm |
| NaCl | 5 gm |
| $K_2HPO_4$ | 2.5 gm |
| Phenol red | 10 mg |

The above components are dissolved in distilled water and the volume of the solution is adjusted to 900 ml.

The pH is adjusted to 7.2 and the medium is autoclaved for 25 minutes.

Glucose (25 g) in 100 ml of distilled water is autoclaved separately for 20 minutes and added aseptically to the medium.

4. Inoculation Medium (14-Liter Fermentor)

| | |
|---|---|
| HySoy® (Humko Sheffield) | 180 gm |
| Amberex® 1003 (Amber) | 90 gm |
| NaCl | 45 gm |
| $K_2HPO_4$ | 22.5 gm |
| *UCON® LB 625 8% solution | 40 ml |

The above components are dissolved in distilled water and the volume of the solution is adjusted to 8 liters.

The pH is adjusted to 7.2 and the medium is autoclaved for 90 minutes. Glucose (225 g) in 1 liter of distilled $H_2O$ is autoclaved separately for 30 minutes and added aseptically to the medium.

5. Production Medium (250-Liter Fermentor)

| | |
|---|---|
| HySoy® (Humko Sheffield) | 3500 gm |
| Amberex® 1003 (Amber) | 1750 gm |
| NaCl | 875 gm |
| $K_2HPO_4$ | 437.5 gm |
| UCON® LB 625 8% solution | 400 ml |

The above components are dissolved in distilled water and the volume is brought up to 165 liters. The pH is adjusted to 7.2 and the medium is autoclaved for 30 minutes.

Glucose (4375 g) in 10 liters of distilled water is autoclaved separately for 30 minutes and added aseptically to the medium.

6. Ouchterlony test for group B

a. A 5 μl sample of Group B streptococcal antiserum is placed in the center well of an Ouchterlony plate (Hyland).

b. A 5 μl sample of streptococcal broth is placed in the outside well.

c. A precipitin reaction occurs if streptococcal Group B cells are present.

7. Gram strain

A rapid method for detecting shape and cell arrangement. The Gram strain reaction will frequently enable the investigator to narrow down the general identification to a small group.

Example 2

Fermentation of type-specific group B *streptococcus* (dialyzed medium)

Following substantially similar procedures as described in Example 1, steps A to C, but substituting for the media used therein the corresponding media described below in Table III, there was obtained type $I_a$, type $I_b$, type II or type III fermentation broth ready for harvesting and isolation.

TABLE III
Dialyzed media for different stages of fermentation
1. Inoculum medium (2-liter flask)

| | |
|---|---|
| HySoy® (Humko Sheffield) | 20 gm |
| Amberex® 1003 (Amber) | 10 gm |
| NaCl | 5 gm |
| $K_2HPO_4$ | 2.5 gm |
| Phenol red | 10 mg |

---

*The UCON® LB 625 8% solution is pre-sterilized for 1 hour.

HySoy® and Amberex® 1003 are dissolved in 50 ml of distilled water and dialyzed with 12,000 to 14,000 molecular weight cut-off. The resulting dialysate together with NaCl, $K_2HPO_4$, and phenol red are diluted with distilled water to a volume of 900 ml.

The pH is adjusted to 7.2 and the medium is autoclaved for 25 minutes.

Glucose (25 g) in 100 ml of distilled water is autoclaved separately for 20 minutes and added aseptically to the medium.

### 2. Inoculation medium (14-liter fermentor)

| | |
|---|---|
| HySoy® (Humko Sheffield) | 180 gm |
| Amberex® 1003 (Amber) | 90 gm |
| NaCl | 45 gm |
| $K_2HPO_4$ | 22.5 gm |
| UCON® LB 625 8% solution | 40 ml |

HySoy® and Amberex® 1003 were dissolved in 450 ml of distilled water and dialyzed with 12,000 to 14,000 molecular weight cut-off. The resulting dialysate together with NaCl, $K_2HPO_4$, and UCON LB 625 are diluted with distilled water to a volume of 8 liters.

The pH is adjusted to 7.2 and the medium is autoclaved for 90 minutes. Glucose (225 g) in 1 liter of distilled $H_2O$ is autoclaved separately for 30 minutes and added aseptically to the medium.

### 3. Production medium (250-liter fermentor)

| | |
|---|---|
| HySoy® (Humko Sheffield) | 3500 gm |
| Amberex® 1003 (Amber) | 1750 gm |
| NaCl | 875 gm |
| $K_2HPO_4$ | 437.5 gm |
| *UCON® LB 625 8% solution | 400 ml |

HySoy® and Amberex® 1003 are dissolved in 9 liters of distilled water and dialyzed with 12,000 to 14,000 molecular weight cut-off. The resulting dialysate together with NaCl, $K_2HPO_4$ and UCON LB 625 are diluted with distilled water to a volume of 165 liters.

The pH is adjusted to 7.2 and the medium is autoclaved for 30 minutes.

Glucose (4375 g) in 10 liters of distilled water is autoclaved separately for 30 minutes and added aseptically to the medium

In Examples 3—6 the following reagents are used:
Acetone, Baker Analyzed
Ammonium Sulfate, Fisher Scientific Co. or Schwarz/Mann
Boric Acid, Merck & Co.
Calcium Chloride, Dihydrate, Baker Analyzed
Cetavlon (Hexadecyltrimethyl-ammonium Bromide), Eastman Kodak—a cationic detergent
DEAE-Cellulose (DE-52), Whatman Ltd.
Ethanol, Denatured (toluene), Merck & Co.
Ethanol, Denatured, 2BA, Type T, Merck & Co.
Ethanol, 200 Proof, U.S. Industrial Chemicals Co.
Glacial Acetic Acid, Merck & Co.
Sepharose 6B, Pharmacia
Sodium Acetate, Anhydrous, Mallinckrodt
Sodium Chloride, Baker Analyzed
Sodium Hydroxide, 50% Solution, Baker Analyzed
Trypsin, Worthington, 238 units/mg.
Reagents are prepared with triply distilled water. Likewise all containers are rinsed in this water.

Example 3

Release Protocol for group B streptococcus type la polysaccharide, product 11059—113
Step 1—Isolation of first crude product

Sixty (60) liters of whole, phenolized broth were charged with 885 gm of calcium chloride, dihydrate to 0.1 M. Ethanol (denatured) was then added to the 44% vol/vol level. The suspension was allowed to stand

---

*The UCON® LB 625 8% solution is pre-sterilized for 1 hour.

for maximal settling of the cells and other insolubles (about 48 hours). The clear supernatant was then transferred into another fractionation vat, with a recovery of 73 liters. Of this volume, 41 liters represent the aqueous phase. On this basis 29 liters of ethanol (denatured) were added to raise the ethanol level to 60%. Again, the suspension was allowed to stand for maximal settling of the precipitate which contains the desired polysaccharide. The clear supernatant was then pumped off and the precipitate collected by centrifugation (Beckman, Model J-21C, in 500 ml cups, for 15 minutes at 6,000 RPM and at 20°C).

Step 2—Extraction of the polysaccharide
The rubbery pellets were suspended with the aid of a blender in 1800 ml of 1% sodium acetate with pH adjustment to 8.5 (using 2 N sodium hydroxide). The suspension was then cleared by centrifugation (as in step 1, except for 30 minutes at 8,000 RPM). There was a firm pellet, overlaid, however, by a creamy paste, which made it necessary to aspirate off the clear supernatant as decanting was not possible. The total volume was 1,240 ml extract.

Step 3—Removal of proteinaceous materials with trypsin
The extract from Step 2 was charged with 30 mg trypsin and incubated at 37°C for 90 minutes. The pH was monitored for the range 8.2—8.6 during the incubation period. The digest was then immediately placed in an ice-bath.

Step 4—Ammonium sulfate precipitation of the polysaccharide
While in an ice-bath the digest was charged with ammonium sulfate (750 gm) to 86% saturation. The resulting suspension was allowed to stand at 4°C overnight to precipitate the polysaccharide. The clear supernatant was removed by aspiration and the salted out material was collected by centrifugation as described in Step 1.

Step 5—Chromatography on DEAE-cellulose
The ammonium sulfate precipitate from Step 4 was dissolved in 200 ml 1% sodium acetate and exhaustively dialyzed for 48 hours (2×12 hours against 10 liters distilled water, followed by 24 hours against 10 liters of 0.05 M borate buffer, pH 8.5). A light turbidity in the retentate was cleared by centrifugation (Sorvall, Model RC-5, in 50 ml cups, at 15,000 RPM for 20 minutes at 4°C). The clear supernatant fluid was then applied to a column of DEAE-Cellulose, equilibrated with sodium borate (0.05 M, pH 8.5), (dimensions of 5.0×30 cm). The column was then washed with 700 ml borate buffer. Sodium chloride (0.3 M) in borate buffer, 800 ml, was then applied to a column to mobilize the adhering polysaccharide. One hundred milliliter (100 ml) fractions were collected and separately assayed serologically for their polysaccharide content. Active fractions (2—8) were pooled and the pool treated with 1.5 volumes of ethanol (denatured) to precipitate the polysaccharide which was recovered after removal of the clear supernatant and by centrifugation (as per Step 1). Pellet volume was estimated as 10 cm$^3$.

Step 6—Recovery of the polysaccharide as a cetavlon complex
The precipitate from Step 5 was resuspended in 300 ml triply distilled water to give a clear solution. One hundred milliliters (100 ml) of a 3% solution of Cetavlon in water was then added with stirring. The polysaccharide aggregated into a single lump allowing its easy removal.

Step 7—Recovery of product intermediate 11059—101
The complex from Step 6 was solubilized in 200 ml 15% sodium acetate, at pH 8.0 at 4°C. It took several hours to achieve complete solubilization. The solution was finally charged with two volumes of ethanol (denatured) to precipitate the polysaccharide as its sodium salt. The precipitate was collected, after removal of the clear supernatant, by centrifugation, as per Step 1. The pellet was triturated with about 100 ml ethanol (denatured) in a blender then collected on a small sintered glass funnel (15 ml, M), washed thereon with 50 ml ethanol and 50 ml acetone, followed by drying in a vacuum desiccator. The product intermediate was called 11059—101, (yield 805 mg).

Step 8—Molecular sieving on Sepharose® 6B and final product recovery
Two hundred milligrams (200 mg) of 11059—101 were dissolved in 12 ml column buffer (1% sodium acetate) and the solution applied to a column of Sepharose® 6B (2.6×90 cm) equilibrated with 1% sodium acetate. Fractions of 12.5 ml were collected and active fractions were identified by the Ouchterlony double immunodiffusion method, using a type specific antiserum. Active fractions (14—29) were pooled. The operation was then repeated with a second 200 mg portion of 11059—101. The 2 pools were combined and concentrated in a Bio Rad No. 80 hollow fiber device to 100 ml. The concentrate was treated with 2 volumes of ethanol (200 proof) to precipitate the polysaccharide. The precipitate was allowed to settle out and recovered, after removal of the supernatant and by centrifugation as per Step 1. The precipitate was then triturated with 50 ml ethanol (200 proof) in a blender and was collected on a small sintered glass funnel (15 ml, M), washed thereon with 50 ml ethanol (200 proof) and 50 ml acetone, followed by drying in a vacuum desiccator to constant weight. Final product: 11059—113 (yield 300 mg).

Example 4
Release protocol for Group B streptococcus type Ib polysaccharide, product 11059—186
Step 1—Preparation of cell-free fermentation broth

Type Ib broth, phenolized to 1% at termination of the fermentation cycle, was cleared of cells by centrifugation in a Sharples ultracentrifuge, Model T-1P.

Step 2—Ultrafiltration/diafiltration of cell-free broth

Sixty liters (60 liters) of the cell-free supernatant were pumped through an ultrafiltration hollow fiber device (Amicon, Model DC-2; molecular weight cut-off of fibers of 50,000 daltons) to a final volume of 11.29 liters.

Step 3—Isolation of first crude product

The concentrate was charged with calcium chloride in 0.1 M and subsequently with ethanol (denatured 2 BA, T type) to 30% by volume. The resulting suspension was centrifuged in a Sharples centrifuge to obtain a clear supernatant (15.13 liters). The ethanol level was then raised to 61% by the addition of 12.06 liters of ethanol (denatured). The resulting precipitate was obtained by allowing it to settle out, removing of most of the clear supernatant, and finally by centrifugation (Beckman, Model J-21C, 50 ml-cups, 15 minutes, 20°C, 6,000 RPM).

Step 4—Isolation of second crude product

The first crude was suspended in 1,800 ml of 3% sodium acetate and full solubilization assured by stirring the suspension vigorously overnight at 4°C. A serological probe performed on a small aliquot of the suspension indicated that the desired polysaccharide is fractionable with ethanol in the 40—60% range. The total volume of the suspension was 1,980 ml. The suspension was accordingly charged with 1,220 ml ethanol (denatured) to the 40% level and the alcoholic suspension cleared by centrifugation in the Beckman as described in Step 3. A considerable amount of insoluble materials were removed at 40% ethanol. The clear supernatant was then charged with additional (1,650 ml) ethanol (denatured) to 60%, and the resulting suspension was allowed to stand for 48 hours to let the insolubles settle out. The insolubles (second crude) were then recovered by removal of most of the supernatant and by centrifugation as already described.

Step 5—Isolation of the polysaccharide as a cetavlon complex

The wet pellets, ca. 40 cc in volume, were dissolved in 250 ml water to give a clear, but pigmented solution. The solution was then dialyzed for 24 hours to remove traces of sodium acetate. The retentate was then charged with 100 ml of a 5% solution of Cetavlon in water. The resulting suspension was let stand at 4°C for 18—20 hours to allow the complexing to go to completion. A small aliquot of the suspension was cleared by centrifugation and the supernatant checked for completion of complexing by adding more Cetavlon to the clear supernatant. There was no further precipitate. The Cetavlon-polysaccharide complex was then collected by centrifugation in a Sorval centrifuge, Model RC-5, in 50 ml cups, for 10 minutes at 15,000 RPM and at 4°C. The pellet was not entirely firm, but with a consistency of molasses allowed of the quick decantation of the clear supernatant.

Step 6—Recovery of the third crude product

The Cetavlon complex was extracted with 200 ml of a 15% solution of sodium acetate at pH 8.0 and the cleared extract (by centrifugation) charged with 2 volumes of ethanol (denatured). A gum formed which settled out overnight, allowing the total decantation of the alcoholic supernatant.

Step 7—De-ionization of the third crude product

The third crude was taken up in 200 ml 0.05 M borate buffer, pH 8.5 and ultrafiltered in an Amicon stirred cell (200 ml capacity, equipped with a 30,000 dalton cut-off membrane). To remove traces of sodium acetate the volume was reduced to 50 ml then increased to 200 ml with borate buffer, followed by reduction to 50 ml.

Step 8—DEAE-cellulose fractionation of the third crude product

The retentate from Step 7 was applied to a column of DEAE-cellulose (5.0×30 cm), equilibrated with 0.5 M borate buffer, pH 8.5. The column was washed with 700 ml borate buffer. The wash did not give a precipitate with 1.5 volumes ethanol, nor did it contain any trace of serologically active material in a test with type specific antiserum (Ib) by the Ouchterlony double immunodiffusion method. The column was then washed with 0.25 M sodium chloride in borate buffer and 100 ml fractions collected. Fractions containing serologically active material (No. 4—10) were pooled, and the polysaccharide was precipitated with 1.5 volumes of ethanol (denatured). After the precipitate had settled out, most of the supernatant was removed by aspiration and the precipitate was finally collected by centrifugation (Sorvall, as described in Step 5). This material was designated the Fourth Crude.

Step 9—Trypsin digestion of fourth crude product to obtain the fifth crude product

The precipitate from Step 8 was dissolved in 150 of a solution of 3% sodium acetate and the pH

**0 038 265**

adjusted down to 8.5. A blender was used to speed up the dispersion of the material. Trypsin was added (20 mg; 238 units/mg), and the system incubated for 90 minutes at 37°C. The pH was monitored from time to time, but found unchanged throughout the incubation cycle. The digest was then charged with 1.5 volumes of ethanol (denatured) and the precipitate collected by centrifugation in a 500 ml cup (Beckman centrifuge). The pellet was triturated in a small blender with absolute ethanol, collected on a small sintered glass funnel (15 ml, M), washed thereon with 50 ml absolute ethanol and 50 ml of acetone and finally dried in a vacuum desiccator overnight. A white powder was obtained, product intermediate (Fifth Crude) 11059—185, with a yield of 741 mg.

Step 10—Purification with ammonium sulfate

Product intermediate 11059—185, as shown by Sepharose® 6B profiling, still carries a considerable amount of contaminant, masking in part the type polysaccharide. It was therefore decided to precipitate the desired polysaccharide with ammonium sulfate, at 86% saturation. The lot of the product intermediate was dissolved in 100 ml triply distilled water, and the solution was charged with enzyme grade ammonium sulfate (61 gm). The resulting suspension was let stand in the cold for 2 hours, and the precipitate was then recovered by centrifugation (Sorvall, 15,000 RPM, 50 ml cups, for 15 minutes at 2°C).

Step 11—Molecular sieving of ammonium sulfate precipitate

The pellets from the previous step were dissolved in 12 ml eluant (1% sodium acetate, pH 7.5). The clear solution, with a pale-yellow pigmentation, was then applied to a column of Sepharose® 6B, equilibrated with 1% sodium acetate, pH 7.5, (dimensions of 2.6×90 cm). Fractions of 12.5 ml were collected, and their polysaccharide content monitored serologically by Ouchterlony double immunodiffusion technique. Active fractions were pooled.

Step 12—Recovery of final product, 11059—186

The pool (213 ml) from Step 11 was diafiltered and concentrated to 20 ml in a suitable Amicon cell, equipped with a 30,000 M. W. cut-off membrane. The concentrate was then charged with 2 volumes of absolute ethanol to precipitate the polysaccharide. This was separated by centrifugation, discarding the supernatant. The pellet was triturated in a small blender with absolute ethanol and the hardened particles collected on a sintered glass funnel. The polysaccharide was washed thereon with 50 ml absolute ethanol and 50 ml acetone and dried in a vacuum desiccator for 24 hours to constant weight. Final product: 11059—186 (yield 189 mg).

Example 5

Release protocol for Group B streptococcus type II polysaccharide, product 11059—176

Step 1—Isolation of first crude product

Eighty (80) liters of whole, phenolized broth were charged with 1,170 gm of calcium chloride, dihydrate, to 0.1 $M$. Ethanol (denatured) was then added to the 40% level (vol/vol). The suspension was allowed to stand for maximal settling of the cells and other insoluble materials (about 48 hours). The clear supernatant was then pumped over into another fractionation vat, with a recovery of 94 liters. The aqueous phase in this system was calculated as 56.4 liters. On this basis, additional 47 liters of ethanol (denatured) were run in with stirring, to an ethanol level of 60%. Again, the suspension was allowed to stand for maximal settling of the precipitate which contains the desired polysaccharide. The clear supernatant was then aspirated off with the aid of a pump, and the precipitate collected by centrifugation (Beckman, Model J-21C, in 500 ml cups, for 15 min. at 6,000 RPM and at 20°C).

Step 2—Isolation of second crude product

The precipitate from Step 1 was resuspended in 1% sodium acetate (3,000 ml), giving a final volume of 3,340 ml and the suspension charged with calcium chloride to 0.1 M. Based on a zerological probe it could be demonstrated that the desired polysaccharide can be fractionated with ethanol in the range of 38—59%. Accordingly, the suspension was charged with 2,050 ml of ethanol (denatured) to the 38% level. The suspension was cleared by centrifugation, as per above, Step 1. Of the total supernatant recovered (4,850 ml) 3,010 ml constitute the aqueous phase. The supernatant was then charged with ethanol (denatured) (2,470 ml) to 59%. A floculent precipitate developed and settled out readily. It was allowed to settle out and the clear supernatant was removed by aspiration. The precipitate was then collected by centrifugation, as already described.

Step 3—Removal of proteinaceous materials with trypsin

The slightly pigmented pellet from Step 2 was dissolved in 500 ml 0.05 M sodium borate, pH 8.5 and the solution was charged with 20 mg trypsin (4,760 unit). The system was incubated at 37°C for 90 minutes.

Step 4—Chromatography on DEAE-cellulose

The trypsin digest from step 3 was applied to a column of DEAE-cellulose (DE-52, Whatman), equilibrated in 0.05 M sodium borate at pH 8.5, (dimension: 5×30 cm). The column was then washed with 700 ml borate buffer. The column was then treated with 800 ml of eluant, containing 0.3 M sodium chloride in borate buffer. Effluent fractions (100 ml) were collected and separately assayed serologically for their polysaccharide content, and for precipitability with 1.5 volumes ethanol. Fraction No. 1 did not give a precipitate, Nos. 4 and 5 had the highest amounts of precipitate, and No. 8 gave only a weak precipitate. Fractions No. 2—7 were pooled and the pool charged with 1.5 volumes of ethanol (denatured) to precipitate the polysaccharide.

Step 5—Isolation of third crude product

The precipitate from step 4 was taken up in 300 ml distilled water, diafiltered to 100 ml, and the process repeated twice more, in each case with replenishing the volume to 300 ml with distilled water. The final retentate of 100 ml was charged with sodium acetate to 1% and the polysaccharide precipitated with 1.5 volumes of ethanol (denatured). The precipitate was collected by allowing it to sediment and by decantating of the clear supernatant. The precipitate was then triturated in a blender with ethanol (denatured) and collected on a small sintered glass funnel (15 ml, N), washed thereon with 50 ml ethanol (denatured) and with 50 ml acetone and dried in a vacuum desiccator. Product intermediate: 11059—160 (yield of 2.654 gm).

Step 6—Cetavlon complexing of the polysaccharide

The lot of product 11059—160 was dissolved in 265 ml triply distilled water to give clear, slightly pigmented solution. The solution was then placed in an ice-bath and while stirred, a solution of 2.65 gm Cetavlon in 50 ml of water was added. The resulting suspension was kept at 0°C for 60 minutes. The Cetavlon-polysaccharide complex was then recovered by centrifugation (Sorvall RC-5, in 50 ml cups, at 2°C, 15,000 RPM, for 40 minutes). The supernatant was carefully decanted from a semi-liquid, viscous pellet. The liquid complex was immediately solubilized in 15% sodium acetate (150 ml) at pH 8.4. The clear solution was then charged with ethanol (denatured) to the 60% level, and the precipitate which formed was allowed to settle out. After total decantation of the supernatant, the precipitate was triturated with ethanol in a blender and collected on a small sintered glass funnel (15 ml, M). It was washed thereon with 50 ml of ethanol (denatured), and with 50 ml acetone, followed by drying in a vacuum desiccator. Product Intermediate: 11059—174 (yield 710 mg).

Step 7—Molecular sieving on Sepharose® 6B and recovery of final product

Four hundred (400) milligrams of product intermediate 11059—174 were dissolved in 12 ml 1% sodium acetate (eluant) and applied to a column of Sepharose® 6B (2.6×90 cm; equilibrated with 1% sodium acetate). Fractions of 12.5 ml were collected and active fractions were identified by the Ouchterlony method, using type-specific antiserum. Fractions 12—30 were pooled and the pool was reduced in volume to 150 ml in an Amicon stirred cell using YM 30 membrane (having a molecular weight cut-off of 30,000 daltons). The concentrate was then transferred into a 600-ml beaker and precipitated with two volumes of ethanol (200 proof). The precipitated polysaccharide was allowed to settle out and the clear supernatant removed by decantating. The sticky settlement was triturated in a blender with ethanol and collected on a small sintered glass funnel (15 ml, M) and washed thereon with 50 ml ethanol and 50 ml acetone. It was then dried in a vacuum desiccator to constant weight. Final Product 11059—176 (yield of 285 mg).

Example 6

Release protocol for group B streptococcus type III polysaccharide, product 11059—176

Step 1—Preparation of cell-free fermentation broth

Type III broth, phenolized to 1% at termination of the fermentation cycle, was cleared of cells by centrifugation in a Sharples ultracentrifuge, model T-1P.

Step 2—Ultrafiltration/diafiltration of cell-free broth

Two hundred (200) liters of the cell-free supernatant from Step 1 were pumped through an ultrafiltration hollow fiber device (Amicon unit, Model DC-2, molecular weight cut-off of 50,000 daltons) to a final volume of 14.9 liters.

Step 3—Isolation of first crude product

The concentrate from Step 2 (14.9 liters) was charged with calcium chloride to 0.1 M and the pH adjusted to 8.2 with 2 N NaOH. It was then charged with 4.95 liters, ethanol (denatured, 2BA T-Type), to 30% by volume. The resulting suspension was centrifuged in a Sharples centrifuge to obtain a clear supernatant (19.0 liters). The aqueous phase of the supernatant was calculated at 14.25 liters. On this basis, the supernatant was charged with additional 21.7 liters ethanol (denatured) to a level of 65%. The resulting suspension was let stand for several days, during which time the desired polysaccharide settled out. The bulk of the clear supernatant was pumped off and the precipitate was collected by centrifugation in 500-ml cups (Beckman, Model J-21C, at 6,000 RPM, 20°C, for 15 minutes). The pellet was triturated in a blender with ethanol (denatured) and collected on a sintered glass funnel (50 ml, M), washed thereon with 50 ml ethanol (denatured) and with 50 ml acetone, followed by drying in a vacuum desiccator overnight. Product 11059—167 (yield 7.22 gm).

13

Step 4—Cetavlon complexing and recovery of second crude product

Seven (7) grams of product intermediate 11059—167 were dissolved in 700 ml ice-cold water and after stirring for one hour the suspension was cleared by centrifugation. The clear supernatant was then charged with 100 ml of a 7.5% solution of Cetavlon and the resulting suspension allowed to stand at 4°C overnight. The insoluble Cetavlon-polysaccharide complex was collected by centrifugation (Sorvall RC-5, in 50-ml cups, at 15,000 RPM, 4°C for 10 min). The supernatant was decanted from a semi-soft, honey-like pellet. The complex was suspended for extraction in 500 ml 15% sodium acetate (no pH adjustment was needed, since it was noted at 8.2). The suspension was stirred for several hours in the cold to assure maximal solubilization. The centrifugation step was repeated to remove some insoluble, pigmented material. The extracted polysaccharide was precipitated from the extract by the addition of ethanol (denatured) to the 61% level. A sticky gum settled out, allowing the supernatant to be decanted totally.

Step 5—Removal of protein by trypsin digestion and recovery of the third crude product

The gum from Step 4 was dissolved in 100 ml of a 3% solution of sodium acetate, with adjustment of the pH to 8.4. The solution was transferred into a 500-ml centrifuge cup, and charged with 20 mg trypsin. The system was then incubated at 37°C for 90 min. with occasional monitoring of the pH. No further pH adjustments needed to be made. At the conclusion of the digest cycle, the polysaccharide was recovered by the addition of 2 volumes of ethanol (denatured). The precipitate was collected by centrifugation (Beckman, Model J21C, at 6,000 RPM, at 20°C and for 10 minutes). The pellet was triturated with ethanol (denatured) in a blender, collected on a small sintered glass funnel (15 ml, M), washed thereon with 50 ml ethanol (denatured) and 50 ml acetone, followed by drying in a vacuum desiccator overnight. Product Intermediate 11059—170 (yield 1,357 mg).

Step 6—Molecular sieving of product intermediate 11059—170

Four hundred (400) milligrams of 11059—170 were dissolved in 12 ml eluant (1% sodium acetate, pH 7.5). The clear solution was then applied to a column of Sepharose® 6B, equilibrated with 1% sodium acetate, pH 7.5, (dimensions of 2.6×90 cm). Fractions of 12.5 ml were collected and their polysaccharide content monitored serologically by the Ouchterlony double immunodiffusion technique. Active fractions (15—32) were pooled and concentrated to ca. 70 ml (Amicon stirred cell, equipped with a 30,000 M. W. cut-off membrane).

Step 7—Recovery of final product

The concentrated pool from Step 6 was then charged with two volumes of 200 proof ethanol. The gummy precipitate which settled out was removed from the precipitation flask and triturated with absolute ethanol in a blender, collected on a small sintered glass funnel (15 ml, M), washed thereon with 50 ml absolute ethanol and 50 ml acetone, followed by drying in a vacuum desiccator to constant weight. Product 11059—179 (yield of 272 mg).

**Claims**

1. A process for the preparation of an antigenic type-specific polysaccharide of Group B *Streptococcus* comprising:

a) growing the Group B *Streptococcus* bacteria types I$_a$, I$_b$, II, or III in a high glucose, soy bean and yeast extract fermentation medium;

b) separating the cell paste from the liquid medium;

c) treating the liquid medium from step b with a strong ionic salt and a water-miscible lower alkanol to precipitate impurities or alternatively digesting the cell paste with an enzyme to obtain a liquid extract;

d) precipitating the crude polysaccharide from the treated liquid medium, or if desired, the liquid extract from digested cell paste with a sufficient amount of a water-miscible lower alkanol;

e) suspending the crude polysaccharide in deionized water and adding thereto sufficient cationic detergent to precipitate the polysaccharide;

f) redissolving the polysaccharide in 15% (wt/wt) sodium acetate aqueous solution;

g) precipitating the semi-purified polysaccharide from the solution with alcohol and, optionally, digesting the resulting precipitate with a proteolytic enzyme, followed by precipitating the enzyme-treated polysaccharide with a sufficient amount of water-miscible alkanol; and

h) eluting the resolubilized semi-purified polysaccharide of step g in a liquid medium through a gel column to obtain the purified polysaccharide.

2. Process according to claim 1 wherein the fermentation medium contains sodium chloride, potassium phosphate dibasic, 2.5% by weight glucose, and the dialyzable constituents of soy bean and yeast extracts.

**Patentansprüche**

1. Verfahren zur Herstellung eines antigenen Typspezifischen Polysaccharids von Streptococcus der Gruppe B, umfassend:

a) Züchten der Streptococcusbakterien der Gruppe B der Typen $I_a$, $I_b$, II oder III in einem stark glucosehaltigen Sojabohnen- und Hefeextrakt-Fermentationsmedium;

b) Abtrennen der Zellpaste von dem flüssigen Medium;

c) Behandeln des flüssigen Mediums der Stufe b) mit einem stark ionischen Salz und einem mit Wasser mischbaren niedrigen Alkanol, um Verunreinigungen auszufällen, oder alternativ Digerieren der Zellpaste mit einem Enzym, um einen flüssigen Extrakt zu erzielen;

d) Ausfällen des rohen Polysaccharids aus dem behandelten flüssigen Medium, oder, falls gewünscht, des flüssigen Extrakts von der digerierten Zellpaste, mit einer ausreichenden Menge eines mit Wasser mischbaren niedrigen Alkanols;

e) Suspendieren des rohen Polysaccharids in entionisiertem Wasser und Zusatz von ausreichend kationischem Detergens, um das Polysaccharid auszufällen;

f) erneutes Auflösen des Polysaccharids in 15% (Gew./Gew.) wässeriger Natriumacetatlösung;

g) Ausfällen des halbgereinigten Polysaccharids aus der Lösung mit Alkohol und gegebenenfalls Digerieren der resultierenden Ausfällung mit einem proteolytischen Enzym, gefolgt von einer Ausfällung des mit Enzym behandelten Polysaccharids mit einer ausreichenden Menge an mit Wasser mischbarem Alkanol; und

h) Eluieren des erneut gelösten halbgereinigten Polysaccharids der Stufe g), in einem flüssigen Medium, durch eine Gelkolonne, zur Erzielung des gereinigten Polysaccharids.

2. Verfahren nach Anspruch 1, bei dem das Fermentationsmedium Natriumchlorid, zweibasisches Kaliumphosphat, 2,5 Gew.-% Glucose und die dialysierbaren Bestandteile von Sojabohnen und Hefeextrakten enthält.

**Revendications**

1. Un procédé pour la préparation d'un polysaccharide antigénique spécifique de type de *Streptococcus* du groupe B comprenant:

a) la culture de bactéries *Streptococcus* du groupe B des types $I_a$, $I_b$, II ou III dans un milieu de fermentation à l'extrait de levure et de soja, à teneur élevée en glucose;

b) La séparation de la pâte de cellule d'avec ls milieu liquide;

c) le traitement du milieu liquide du stade avec un sel ionique fort et un alcanol inférieur miscible à l'eau pour précipiter les impuretés, ou sinon la digestion de la pâte de cellule avec une enzyme pour obtenir un extrait liquide;

d) la précipitation du polysaccharide brut du milieu liquide traité ou, si on le désire, de l'extrait liquide de la pâte de cellule digérée, avec une quantité suffisante d'un alcanol inférieur miscible à l'eau;

e) la mise en suspension du polysaccharide brut dans de l'eau désionisée et l'addition d'un détergent cationique suffisant pour précipiter le polysaccharide;

f) la redissolution du polysaccharide dans une solution aqueuse d'acétate de sodium à 15% (p/p);

g) la précipitation du polysaccharide semi-purifié dans la solution avec de l'alcool, et éventuellement la digestion du précipité obtenu avec une enzyme protéolytique, suivie de la précipitation du polysaccharide traité par une enzyme avec une quantité suffisante d'un alcanol miscible à l'eau; et

h) l'élution du polysaccharide semi-purifié resolubilisé du stade g dans un milieu liquide à travers une colonne de gel pour obtenir le polysaccharide purifié.

2. Procédé selon la revendication 1, dans lequel le milieu de fermentation contient du chlorure de sodium, du phosphate dipotassique, 2,5% en poids de glucose et les constituants dialysables de soja et de l'extrait de levure.